(19) Europäisches Patentamt
European Patent Office
Office européen des brevets

(11) EP 1 258 248 A2

(12) # EUROPEAN PATENT APPLICATION

(43) Date of publication:
20.11.2002 Bulletin 2002/47

(51) Int Cl.[7]: **A61K 33/24**, A61P 35/00, A61K 31/70, A61K 31/335

(21) Application number: 02008178.2

(22) Date of filing: 16.04.2002

(84) Designated Contracting States:
**AT BE CH CY DE DK ES FI FR GB GR IE IT LI LU MC NL PT SE TR**
Designated Extension States:
**AL LT LV MK RO SI**

(30) Priority: 18.05.2001 US 860383

(71) Applicant: **TAP Pharmaceutical Products, Inc.**
**Lake Forest, Illinois 60045 (US)**

(72) Inventor: **Smith Dordal, Margaret Ann**
**Lake Forest, Illinois 60045 (US)**

(74) Representative: **Modiano, Guido, Dr.-Ing. et al**
**Modiano, Josif, Pisanty & Staub,**
**Baaderstrasse 3**
**80469 München (DE)**

(54) **Tumor treatments comprising a fumagillol derivative and a further antineoplastic agent**

(57) A method of and a kit for treating a tumor by administering a therapeutically-effective amount of a fumagillol derivative and at least one antineoplastic agent to a patient in need of such treatment is disclosed. A presently preferred treatment is to administer 6-O-(N-chloroacetylcarbamoyl) fumagillol and paclitaxel. A method of and a kit for treating a tumor by administering a therapeutically-effective amount of a fumagillol derivative, at least one platinum complex and at least one other antineoplastic agent to a patient in need of such treatment is also disclosed. A presently preferred treatment is to administer 6-O-(N-chloroacetylcarbamoyl) fumagillol, carboplatin and paclitaxel.

EP 1 258 248 A2

## Description

### Field of the Invention

The invention is directed to a method of and a kit for treating a tumor by administering a therapeutically-effective amount of a fumagillol derivative and at least one antineoplastic agent to a patient in need of such treatment. A presently preferred treatment is to administer 6-O-(N-chloroacetylcarbamoyl) fumagillol and paclitaxel. The invention is also directed to a method of and a kit for treating a tumor by administering a therapeutically-effective amount of a fumagillol derivative, at least one platinum complex and at least one other antineoplastic agent to a patient in need of such treatment. A presently preferred treatment is to administer 6-O-(N-chloroacetylcarbamoyl) fumagillol, carboplatin and paclitaxel.

### Background of the Invention

Pharmacotherapies for cancer have mainly employed chemotherapeutic agents to kill tumor cells by cytocidal activity. Unfortunately, to kill tumor cells, such chemotherapeutic agents must be given in doses which are also highly toxic to normal cells.

Angiogenesis inhibitors have been proposed as alternatives or adjuncts to the conventional cancer treatments. Angiogenesis, the induction of growth of new capillary blood vessels, is important in normal processes such as development of the embryo, formation of the corpus luteum and healing of wounds. It is also an important component in pathological processes such as chronic inflammation, certain immune responses, and neoplasia.

Angiogenesis is essential for the growth of solid tumors, as the growth of vascular endothelial cells is extremely vigorous in tumor tissues. It is now accepted that angiogenesis is induced by most malignant tumors and that it is necessary for their continued growth and survival. It is also recognized that angiogenesis is a major component of a number of ophthalmological pathologies including diabetic retinopathy, retrolental fibroplasia and neovascular glaucoma. Additionally, angiogenesis is now recognized as a major component in other non-neoplastic pathological conditions including rheumatoid arthritis (in which abnormal capillary growth can destroy joint cartilage), hemanogiomas (in which abnormal capillary proliferation appears in newborns and can persist for up to two years) and psoriasis, in which excessive proliferation and shedding may be dependent on abnormal capillary growth in the dermis.

The realization that tumors, as well as many non-neoplastic diseases, are angiogenesis-dependent has led to a search for angiogenesis inhibitors that might be used therapeutically.

One promising drug is the natural product fumagillin, and related fumagillol derivatives. Fumagillin has been disclosed to be an angiogenesis inhibitor in U.S. Patent No. 5,135,919. Moreover, various fumagillol derivatives have been disclosed to be angiogenesis inhibitors in U.S. Patent Nos. 5,180,738; 5,164,410; 5,196,406; 5,166,172; and 5,290,807. In particular, one fumagillol derivative (*3R,4S,5S,6R*)-5-methoxy-4-(*2R,3R*)-2-methyl-3-(3-methyl-2-butenyl)-oxiranyl)-1-oxaspiro(2,5)oct-6-yl(chloroacetyl) carbamate, also known as 6-O-(N-chloroacetylcarbamoyl) fumagillol or TNP-470 ( available from Takeda Chemical Industries, Ltd. of Japan) is a particularly potent anti-angiogenesis compound. Bhargava *et al.* review TNP-470 in Chapter 26 of Angiogenesis in Health and Disease, G. M. Rubanyi, ed., Marcel/Dekkker: 2000, pp. 387-406.

To enhance the activity of anti-angiogenesis treatments, use of adjunct treatments has been explored. In particular, fumagillin and fumagillol derivatives such as TNP-470 have been tested in conjunction with treatment with various other drugs to enhance efficacy for treatment of diseases induced by abnormally stimulated neovascularization, such as inflammatory diseases (rheumatism and psoriasis among others), diabetic retinopathy and cancer.

For example, EP 0 658 342 B1 discloses use of a fumagillol derivative and a platinum complex; treatment with adriamycin (doxorubicin hydrochloride) and TNP-470 is disclosed by Kamei *et al.* in The Journal of Pharmacology and Experimental Therapeutics, Vol. 264, pp. 469-474 (1993); treatment with TNP-470 and bromocriptine is disclosed by Takechi *et al.* in Anticancer Research, Vol. 14, pp. 157-162 (1994); treatment with TNP-470 and cytoxan is disclosed by Teicher *et al.* in Anticancer Research, Vol. 13, pp. 2101-2106 (1993); treatment of non-small cell lung cancer with paclitaxel and TNP-470 is disclosed by Satoh *et al.* in Anticancer Research, Vol. 18, pp. 1027-1030 (1998); treatment of Lewis lung carcinoma with paclitaxel, carboplatin, minocycline and TNP-470 is disclosed by Herbst *et al.* in Cancer Chemother. Pharmacol., Vol. 41, pp. 497-504 (1998) and treatment with fumagillin and heparin or sulfated cyclodextrins is disclosed in U.S. Patent No. 5,135,919.

Despite the existence of animal studies indicating that the combination of an angiogenesis inhibitor such as fumagillin or its analogues with cytocidal chemotherapy might result in increased anti-tumor activity (as exemplified by the references listed in the previous paragraph *inter alia),* one skilled in the art of chemotherapy could not assume that the same treatment would have the same effect in humans. This is so as other angiogenesis inhibitors have failed to show activity in humans despite a demonstration of activity in animal or in *in vitro* studies.

For example, matrix metalloproteinase inhibitors of angiogenesis including prinomastat (AG3340), batimastat,

marimastat and others have increased the survival rate for animals in experimental models of lung cancer and other tumors (see Shalinsky *et al.*, "Broad Anti-Tumor and Antiangiogenic Activities of AG3340, A Potent and Selective MMP Inhibitor Undergoing Advanced Oncology Clinical Trials" in Ann. N.Y. Acad. Sci. June 30, (878), pp. 236-270, (1999); Scatena, "Prinomastat, A Hydroxamate-Based Matrix Metalloproteinase Inhibitor. A Novel Pharmacological Approach For Tissue Remodelling-Related Diseases" in Expert Opin. Investig. Drugs, Sept. 9, (9), pp. 2159-2165, (2000); Johnston, M. R., "Validation of an Orthotopic Model of Human Lung Cancer with Regional and Systemic Metastases" in Ann. Thorac. Surg. April, 71, (4) pp. 1120-1125, (2001) and Maekawa *et al.*, "Anti-Metastatic Efficacy and Safety of MMI-166, A Selective Matrix Metalloproteinase Inhibitor" in Clin. Exp. Metastasis, 18(1), pp. 61-66 (2000) *inter alia).*

Nevertheless, clinical trials of prinomastat and marimastat have neither improved patient survival, nor increased the rate of tumor response in comparison to a placebo, even when administered in combination with other chemotherapies, and have been halted (see; Shepard *et al.,* Proc. ASCO 2001; 20 (11); Ahmann *et al.,* Proc. ASCO 2001; 20 (692) and Scrip 2617 Review Issue 2000, p. 66, Feb. 14, 2001 (200102140) "New Products Under Fire", *inter alia).*

This failure of drugs active in animal studies to improve the survival rate, or rate of tumor response in humans is well known to one skilled in the art of chemotherapy, and is not limited to drugs that inhibit angiogenesis. For example, menogaril had broad activity against cancers in mice, including leukemia, breast and pancreas (McGovren *et al.,* Invest. New Drugs, 2(4), pp. 359-367 (1984); Yoshida *et al.,* Anticancer Res., May-June, 16 (3A), pp. 1155-1159, (1996); and Sternberg *et al.,* Am. J. Clin. Oncol., June, 10 (3), pp. 219-221 (1987)) but did not have significant activity when tested in human patients with multiple myeloma or lymphocytic leukemia (Kucuk *et al.,* Am. J. Clin. Oncol., August, 23 (4), pp.379-383, (2000)), breast cancer (Long *et al.,* Am. J. Clin. Oncol., Oct., 11 (5), pp. 524-527, (1988)) or pancreatic cancer (Sternberg *et al.*, Am. J. Clin. Oncol., April, 11 (2), pp.174-176, (1988)).

Therefore, enhanced therapies for treating tumors in humans with fumagillol derivatives such as TNP-470, in conjunction with administration of other drugs, are still desirable.

## Brief Summary of the Invention

The invention is directed to a method of treating a tumor comprising the step of administering a therapeutically-effective amount of

a fumagillol derivative; and,
at least one antineoplastic agent selected from the group consisting of paclitaxel, gemcitabine and carmustin;
to a patient in need of such treatment.

For the practice of the method, the fumagillol derivative and the antineoplastic agent may be administered sequentially. The fumagillol derivative may be 6-O-(N-chloroacetylcarbamoyl) fumagillol and the antineoplastic agent may be paclitaxel. In one regimen of the above method, 6-O-(N-chloroacetylcarbamoyl) fumagillol may be administered three times a week and paclitaxel may be administered once every three weeks. For this regimen, a presently preferred dosage range is 30-60 mg/m$^2$ 6-O-(N-chloroacetylcarbamoyl) fumagillol and 175-225 mg/m$^2$ paclitaxel. In another regimen of the above method, 135-175 mg/m$^2$ 6-O-(N-chloroacetylcarbamoyl) fumagillol may be administered once a week and 90-100 mg/m$^2$ paclitaxel may be administered once a week.

The invention is also directed to a method of treating a tumor comprising the step of administering a therapeutically-effective amount of

a fumagillol derivative;
at least one antineoplastic agent selected from the group consisting of paclitaxel, gemcitabine and carmustine; and
at least one platinum complex;
to a patient in need of such treatment.

In this method, the platinum complex may be cisplatin, carboplatin, nedaplatin, zeniplatin, enloplatin, lobaplatin, ormaplatin, oxaliplatin, loboplatin or sebriplatin. Routinely the fumagillol derivative, the platinum complex and the antineoplastic agent may be administered sequentially. A presently preferred antineoplastic agent is paclitaxel, a presently preferred fumagillol derivative is 6-O-(N-chloroacetylcarbamoyl) fumagillol and a presently preferred platinum complex is carboplatin. In one regimen of the above method, paclitaxel may be administered once every three weeks; 6-O-(N-chloroacetylcarbamoyl) fumagillol may be administered three times a week, and carboplatin may be administered once every three weeks, in a three-week cycle. For this regimen, a presently preferred dosage range is 175-225 mg/m$^2$ paclitaxel, 30-60 mg/m$^2$ 6-O-(N-chloroacetylcarbamoyl) fumagillol, and carboplatin at a dose of AUC 5-6; and a presently most preferred dosage is 225 mg/m$^2$ paclitaxel, 60 mg/m$^2$ 6-O-(N-chloroacetylcarbamoyl) fumagillol and carboplatin at a dose of AUC 5. In another regimen of the above method, 175-225 mg/m$^2$ paclitaxel may be administered

once every three weeks, 2.5-10 mg/m$^2$ 6-O-(N-chloroacetylcarbamoyl) fumagillol may be administered continuously, and carboplatin at a dose of AUC 5-6 may be administered once every three weeks, in a three-week cycle. Presently most preferred dosages for this regimen are 225 mg/m$^2$ paclitaxel, 2.5-10 mg/m$^2$ 6-O-(N-chloroacetylcarbamoyl) fumagillol and carboplatin at a dose of AUC 5.

The invention is also directed to a kit comprising:

a fumagillol derivative, optionally in a pharmaceutically acceptable carrier or excipient; and,
at least one antineoplastic agent selected from the group consisting of paclitaxel, gemcitabine and carmustin, optionally in a pharmaceutically acceptable carrier or excipient.

In the kit, the fumagillol derivative may be 6-O-(N-chloroacetylcarbamoyl) fumagillol and the antineoplastic agent may be paclitaxel.

The invention is also directed to another kit comprising:

a fumagillol derivative, optionally in a pharmaceutically acceptable carrier or excipient;
at least one antineoplastic agent selected from the group consisting of paclitaxel, gemcitabine and carmustine, optionally in a pharmaceutically acceptable carrier or excipient; and
at least one platinum complex, optionally in a pharmaceutically acceptable carrier or excipient.

The platinum complex may be cisplatin, carboplatin, nedaplatin, zeniplatin, enloplatin, lobaplatin, ormaplatin, oxaliplatin, loboplatin or sebriplatin. In this kit, a presently preferred antineoplastic agent is paclitaxel, a presently preferred fumagillol derivative is 6-O-(N-chloroacetylcarbamoyl) fumagillol and a presently preferred platinum complex is carboplatin.

## Detailed Description of the Invention

The invention is directed to a method of and a kit for treating a tumor by administering a therapeutically-effective amount of a fumagillol derivative and at least one antineoplastic agent to a patient in need of such treatment. A presently preferred treatment is to administer 6-O-(N-chloroacetylcarbamoyl) fumagillol and paclitaxel. The invention is also directed to a method of and a kit for treating a tumor by administering a therapeutically-effective amount of a fumagillol derivative, at least one platinum complex and at least one other antineoplastic agent to a patient in need of such treatment. A presently preferred treatment is to administer 6-O-(N-chloroacetylcarbamoyl) fumagillol, carboplatin and paclitaxel.

The methods and kits are described in greater detail below.

### *Definitions of Terms*

As used herein, the term "angiogenesis" refers to the development of blood vessels.

As used herein, the term "tumor" refers to brain tumors including neuroblastoma, medulloblastoma, meningioma and glioblastoma; head and neck cancer, thyroid carcinoma, endocrine tumors, esophageal cancer, small cell and non-small cell lung cancer, colon cancer, rectal cancer, pancreatic cancer, gastric cancer, bladder cancer, hepatic cancer, malignant lymphoma, acute and chronic leukemia, Kaposi's sarcoma, glioma, hemangioma, osteosarcoma, soft tissue sarcoma, malignant melanoma, skin cancer, prostate cancer, breast carcinoma, choriocarcinoma, ovarian cancer, cervical cancer, uterine cancer and mesenchymal tumors among others.

As used herein, the term "patient" refers to a human in need of the treatment to be administered.

AUC as used herein refers to "area under the curve", and is calculated according to the formula

$$\text{Dose (mg)} = \text{AUC} \times (\text{glomerular filtration rate} + 25)$$

Therefore, AUC 6 (for example) refers to a dose calculated to provide an exposure of 6 mg/mL/min.

Glomerular filtration rate was estimated by creatinine clearance, either measured or calculated from the patient's age, weight, gender and serum creatinine according to the Cockcroft-Gault equation

$$\text{Cr CL} = ((140\text{-age}) \times \text{weight (kg)}/72 \times \text{serum creatinine}) \times 1.0 \text{ if male or}$$

$$0.85 \text{ if female}$$

The term "alkyl" as used herein, alone or in combination, refers to $C_1$-$C_{12}$ straight or branched, substituted or unsubstituted saturated chain radicals derived from saturated hydrocarbons by the removal of one hydrogen atom, unless the term alkyl is preceded by a $C_x$-$C_y$ designation. Representative examples of alkyl groups include methyl, ethyl, n-propyl, iso-propyl, n-butyl, sec-butyl, iso-butyl, and tert-butyl among others.

The term "alkenyl" as used herein, alone or in combination, refers to a substituted or unsubstituted straight-chain or substituted or unsubstituted branched-chain alkenyl radical containing from 2 to 10 carbon atoms. Examples of such radicals include, but are not limited to, ethenyl, E- and Z-pentenyl, decenyl and the like.

The term "alkynyl" as used herein, alone or in combination, refers to a substituted or unsubstituted straight or substituted or unsubstituted branched chain alkynyl radical containing from 2 to 10 carbon atoms. Examples of such radicals include, but are not limited to ethynyl, propynyl, propargyl, butynyl, hexynyl, decynyl and the like.

The term "lower" modifying "alkyl", "alkenyl", "alkynyl" or "alkoxy" refers to a $C_1$-$C_6$ unit for a particular functionality. For example lower alkyl means $C_1$-$C_6$ alkyl.

The term "aliphatic acyl" as used herein, alone or in combination, refers to radicals of formula alkyl-C(O)-, alkenyl-C(O)- and alkynyl-C(O)- derived from an alkane-, alkene- or alkyncarboxylic acid, wherein the terms "alkyl", "alkenyl" and "alkynyl" are as defined above. Examples of such aliphatic acyl radicals include, but are not limited to, acetyl, propionyl, butyryl, valeryl, 4-methylvaleryl, acryloyl, crotyl, propiolyl and methylpropiolyl, among others.

The term "cycloalkyl" as used herein refers to an aliphatic ring system having 3 to 10 carbon atoms and 1 to 3 rings, including, but not limited to cyclopropyl, cyclopentyl, cyclohexyl, norbornyl, and adamantyl among others. Cycloalkyl groups can be unsubstituted or substituted with one, two or three substituents independently selected from lower alkyl, haloalkyl, alkoxy, thioalkoxy, amino, alkylamino, dialkylamino, hydroxy, halo, mercapto, nitro, carboxaldehyde, carboxy, alkoxycarbonyl and carboxamide. "Cycloalkyl" includes cis or trans forms. Furthermore, the substituents may either be in endo or exo positions in the bridged bicyclic systems.

The term "cycloalkenyl" as used herein alone or in combination refers to a cyclic carbocycle containing from 4 to 8 carbon atoms and one or more double bonds. Examples of such cycloalkenyl radicals include, but are not limited to, cyclopentenyl, cyclohexenyl, cyclopentadienyl and the like.

The term "cycloalkylalkyl" as used herein refers to a cycloalkyl group appended to a lower alkyl radical, including, but not limited to cyclohexylmethyl.

The term "halo" or "halogen" as used herein refers to I, Br, Cl or F.

The term "haloalkyl" as used herein refers to a lower alkyl radical, to which is appended at least one halogen substituent, for example chloromethyl, fluoroethyl, trifluoromethyl and pentafluoroethyl among others.

The term "alkoxy" as used herein, alone or in combination, refers to an alkyl ether radical, wherein the term "alkyl" is as defined above. Examples of suitable alkyl ether radicals include, but are not limited to, methoxy, ethoxy, n-propoxy, iso-propoxy, n-butoxy, iso-butoxy, sec-butoxy, tert-butoxy and the like.

The term "alkenoxy" as used herein, alone or in combination, refers to a radical of formula alkenyl-O-, provided that the radical is not an enol ether, wherein the term "alkenyl" is as defined above. Examples of suitable alkenoxy radicals include, but are not limited to, allyloxy, E- and Z- 3-methyl-2-propenoxy and the like.

The term "alkynoxy" as used herein, alone or in combination, refers to a radical of formula alkynyl-O-, provided that the radical is not an -ynol ether. Examples of suitable alkynoxy radicals include, but are not limited to, propargyloxy, 2-butynyloxy and the like.

The term "carboxyl" as used herein refers to a carboxylic acid radical, -C(O)OH.

The term "carboxy" as used herein refers to -C(O)-.

The term "thioalkoxy" refers to a thioether radical of formula alkyl-S-, wherein "alkyl" is as defined above.

The term "sulfonamido" as used herein refers to $-SO_2NH_2$.

The term "carboxaldehyde" as used herein refers to -C(O)R wherein R is hydrogen.

The terms "carboxamide" or "amide" as used herein refer to $-C(O)NR_aR_b$ wherein $R_a$ and $R_b$ are each independently hydrogen, alkyl or any other suitable substituent.

The term "alkoxyalkoxy" as used herein refers to $R_cO-R_dO-$ wherein $R_c$ is lower alkyl as defined above and $R_d$ is alkylene wherein alkylene is $-(CH_2)_{n'}-$ wherein n' is an integer from 1 to 6. Representative examples of alkoxyalkoxy groups include methoxymethoxy, ethoxymethoxy, t-butoxymethoxy among others.

The term "alkylamino" as used herein refers to $R_eNH-$ wherein $R_e$ is a lower alkyl group, for example, ethylamino, butylamino, among others.

The term "alkenylamino", as used herein, alone or in combination, refers to a radical of formula alkenyl-NH- or $(alkenyl)_2N-$, wherein the term "alkenyl" is as defined above, provided that the radical is not an enamine. An example of such alkenylamino radical is the allylamino radical.

The term "alkynylamino" as used herein, alone or in combination, refers to a radical of formula alkynyl-NH- or $(alkynyl)_2N-$ wherein the term "alkynyl" is as defined above, provided that the radical is not an amine. An example of such alkynylamino radicals is the propargyl amino radical.

The term "dialkylamino" as used herein refers to $R_fR_gN-$ wherein $R_f$ and $R_g$ are independently selected from

lower alkyl, for example diethylamino, and methyl propylamino, among others.

The term "amino" as used herein refers to $H_2N$-.

The term "alkoxycarbonyl" as used herein refers to an alkoxyl group as previously defined appended to the parent molecular moiety through a carbonyl group. Examples of alkoxycarbonyl include methoxycarbonyl, ethoxycarbonyl, and isopropoxycarbonyl among others.

The term "aryl" or "aromatic" as used herein alone or in combination refers to a substituted or unsubstituted carbocyclic aromatic group having about 6 to 12 carbon atoms such as phenyl, naphthyl, indenyl, indanyl, azulenyl, fluorenyl and anthracenyl; or a heterocyclic aromatic group which is an aromatic ring containing at least one endocyclic N, O or S atom such as furyl, thienyl, pyridyl, pyrrolyl, oxazolyl, thiazolyl, imidazolyl, pyrazolyl, 2-pyrazolinyl, pyrazolidinyl, isoxazolyl, isothiazolyl, 1,2,3-oxadiazolyl, 1,2,3-triazolyl, 1,3,4-thiadiazolyl, pyridazinyl, pyrimidinyl, pyrazinyl, 1,3,5-triazinyl, 1,3,5-trithianyl, indolizinyl, indolyl, isoindolyl, 3H-indolyl, indolinyl, benzo[b]furanyl, 2,3-dihydrobenzofuranyl, benzo[b]thiophenyl, 1H-indazolyl, benzimidazolyl, benzthiazolyl, purinyl, 4H-quinolizinyl, isoquinolinyl, cinnolinyl, phthalazinyl, quinazolinyl, quinoxalinyl, 1,8-naphthridinyl, pteridinyl, carbazolyl, acridinyl, phenazinyl, phenothiazinyl, phenoxyazinyl, pyrazolo[1,5-c]triazinyl and the like. "Arylalkyl" and "alkylaryl" employ the term "alkyl" as defined above. Rings may be multiply substituted.

The term "aralkyl" as used herein, alone or in combination, refers to an aryl substituted alkyl radical, wherein the terms "alkyl" and "aryl" are as defined above. Examples of suitable aralkyl radicals include, but are not limited to, phenylmethyl, phenethyl, phenylhexyl, diphenylmethyl, pyridylmethyl, tetrazolyl methyl, furylmethyl, imidazolyl methyl, indolylmethyl, thienylpropyl and the like.

The term "aralkenyl" as used herein, alone or in combination, refers to an aryl substituted alkenyl radical, wherein the terms "aryl" and "alkenyl" are as defined above.

The term "arylamino" as used herein, alone or in combination, refers to a radical of formula aryl-NH-, wherein "aryl" is as defined above. Examples of arylamino radicals include, but are not limited to, phenylamino(anilido), naphthlamino, 2-, 3-, and 4- pyridylamino and the like.

The term "biaryl" as used herein, alone or in combination, refers to a radical of formula aryl-aryl, wherein the term "aryl" is as defined above.

The term "thioaryl" as used herein, alone or in combination, refers to a radical of formula aryl-S-, wherein the term "aryl" is as defined above. An example of a thioaryl radical is the thiophenyl radical.

The term "aroyl" as used herein, alone or in combination, refers to a radical of formula aryl-CO-, wherein the term "aryl" is as defined above. Examples of suitable aromatic acyl radicals include, but are not limited to, benzoyl, 4-halobenzoyl, 4-carboxybenzoyl, naphthoyl, pyridylcarbonyl and the like.

The term "heterocyclyl" as used herein, alone or in combination, refers to a non-aromatic 3- to 10- membered ring containing at least one endocyclic N, O, or S atom. The heterocycle may be optionally aryl-fused. The heterocycle may also optionally be substituted with at least one substituent which is independently selected from the group consisting of hydrogen, halogen, hydroxyl, amino, nitro, trifluoromethyl, trifluoromethoxy, alkyl, aralkyl, alkenyl, alkynyl, aryl, cyano, carboxy, carboalkoxy, carboxyalkyl, oxo, arylsulfonyl and aralkylaminocarbonyl among others.

The term "heterocycloyl", as used herein refers to radicals of formula heterocyclyl-C(O)-, wherein the term "heterocyclyl" is as defined above. Examples of suitable heterocycloyl radicals include tetrahydrofuranylcarbonyl, piperidinecarbonyl and tetrahydrothiophenecarbonyl among others.

The term "alkylheterocyclyl" as used herein refers to an alkyl group as previously defined appended to the parent molecular moiety through a heterocyclyl group.

The term "heterocyclylalkyl" as used herein refers to a heterocyclyl group as previously defined appended to the parent molecular moiety through an alkyl group.

The term "amirial" as used herein refers to a hemi-acetal of the structure $R_hC(NR_iR_j)(NR_kR_l)$- wherein $R_h$, $R_i$, $R_j$, $R_k$ and $R_l$ are each independently hydrogen, alkyl or any other suitable substituent.

The term "amide" as used herein refers to a moiety ending with a $-C(O)NH_2$ functional group.

The term "ester" as used herein refers to $-C(O)R_m$, wherein $R_m$ is hydrogen, alkyl or any other suitable substituent.

The term "carbamate" as used herein refers to compounds based on carbamic acid, $NH_2C(O)OH$.

Use of the above terms is meant to encompass substituted and unsubstituted moieties. Substitution may be by one or more groups such as alcohols, ethers, esters, amides, sulfones, sulfides, hydroxyl, nitro, cyano, carboxy, amines, heteroatoms, lower alkyl, lower alkoxy, lower alkoxycarbonyl, alkoxyalkoxy, acyloxy, halogens, trifluoromethoxy, trifluoromethyl, alkyl, aralkyl, alkenyl, alkynyl, aryl, cyano, carboxy, carboalkoxy, carboxyalkyl, cycloalkyl, cycloalkylalkyl, heterocyclyl, alkylheterocyclyl, heterocyclylalkyl, oxo, arylsulfonyl and aralkylaminocarbonyl or any of the substituents of the preceding paragraphs or any of those substituents either attached directly or by suitable linkers. The linkers are typically short chains of 1-3 atoms containing any combination of -C-, -C(O)-, -NH-, -S-, -S(O)-, -O-, -C(O)O- or -S(O)O-. Rings may be substituted multiple times.

The terms "electron-withdrawing" or "electron-donating" refer to the ability of a substituent to withdraw or

donate electrons relative to that of hydrogen if hydrogen occupied the same position in the molecule. These terms are well-understood by one skilled in the art and are discussed in Advanced Organic Chemistry by J. March, 1985, pp. 16-18, incorporated herein by reference. Electron withdrawing groups include halo, nitro, carboxyl, lower alkenyl, lower alkynyl, carboxaldehyde, carboxyamido, aryl, quaternary ammonium, trifluoromethyl, and aryl lower alkanoyl among others. Electron donating groups include such groups as hydroxy, lower alkyl, amino, lower alkylamino, di(lower alkyl) amino, aryloxy, mercapto, lower alkylthio, lower alkylmercapto, and disulfide among others. One skilled in the art will appreciate that the aforesaid substituents may have electron donating or electron withdrawing properties under different chemical conditions. Moreover, the present invention contemplates any combination of substituents selected from the above-identified groups.

The most preferred electron donating or electron withdrawing substituents are halo, nitro, alkanoyl, carboxaldehyde, arylalkanoyl, aryloxy, carboxyl, carboxamide, cyano, sulfonyl, sulfoxide, heterocyclyl, guanidine, quaternary ammonium, lower alkenyl, lower alkynyl, sulfonium salts, hydroxy, lower alkoxy, lower alkyl, amino, lower alkylamino, di(lower alkyl)amino, amine lower alkyl mercapto, mercaptoalkyl, alkylthio and alkyldithio.

Asymmetric centers may exist in the compounds of the present invention. Except where otherwise noted, the present invention contemplates the various stereoisomers and mixtures thereof. Accordingly, whenever a bond is represented by a wavy line, it is intended that a mixture of stereo-orientations or an individual isomer of assigned or unassigned orientation may be present.

*The Kits*

The components (or active ingredients) for the treatment can be assembled and used as a kit. Each of the separate components of the kit may be formulated into a separate composition. Each composition of each individual component may include, in addition to the active ingredient, a pharmaceutically acceptable carrier, excipient or diluent, and can be administered individually. The method of administration is described in greater detail in the Method section below. The present invention includes kits of at least two components as well as kits of at least three components. Each type of kit will be described in greater detail below.

*Two-Component Kits*

A kit of the present invention includes at least two components: a fumagillol derivative and at least one antineoplastic agent.

The ratio of fumagillol to antineoplastic agent is not critical, and will depend upon the particular patient and tumor to be treated.

Fumagillin is a compound of Formula I, shown below.

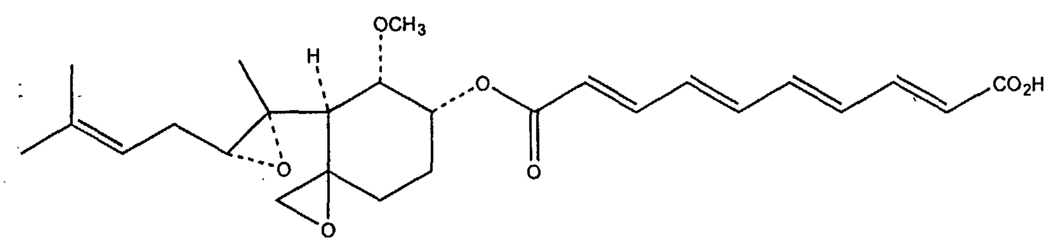

Formula I

Fumagillol derivatives include compounds of general Formula II shown below.

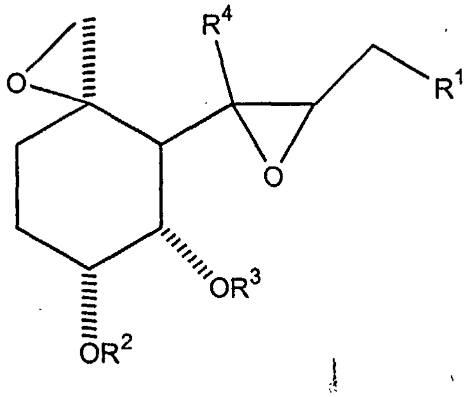

Formula II

wherein $R^1$ and $R^4$ are each independently selected from the group consisting of hydrogen, halogen, hydroxyl, alkyl, alkenyl, alkynyl, alkoxy, alkenoxy, alkynoxy, thioalkoxy, hydroxyalkyl, aliphatic acyl, $-CF_3$, $-NO_2$, $-NH_2$, -CN, $-CO_2H$, -SH, carboxy, $-N(C_1-C_3$ alkyl$)-C(O)(C_1-C_3$ alkyl), $-NHC(O)NH(C_1-C_3$ alkyl), $-NHC(O)N(C_1-C_3$ alkyl)C(O)NH $(C_1-C_3$ alkyl), $-NHSO_2(C_1-C_3$ alkyl), $-NHSO_2$(aryl), $-C_1-C_3$ alkylamino, alkenylamino, alkynylamino, di($C_1-C_3$ alkyl)amino, $-C(O)O-(C_1-C_3$ alkyl), $-C(O)NH-(C_1-C_3$ alkyl), -CH=NOH, $-PO_3H_2$, $-OPO_3H_2$, $-C(O)N(C_1-C_3$ alkyl$)_2$, haloalkyl, alkoxyalkoxy, alkoxyalkyl, alkoxycarbonyl, carboxaldehyde, carboxamide, cycloalkyl, cycloalkenyl, cycloalkynyl, cycloalkylalkyl, aryl, aroyl, aryloxy, arylamino, biaryl, thioaryl, diarylamino, heterocyclyl, alkylaryl, aralkenyl, aralkyl, alkylheterocyclyl, heterocyclylalkyl, heterocycloyl, sulfonyl, $-SO_2-(C_1-C_3$ alkyl), $-SO_3-(C_1-C_3$ alkyl), sulfonamido, aryloxyalkyl, carbamate and -C(O)NH(benzyl);

and $R^2$ and $R^3$ are each independently selected from the group consisting of hydrogen, alkyl, alkenyl, alkynyl, alkoxy, alkenoxy, alkynoxy, thioalkoxy, hydroxyalkyl, aliphatic acyl, $-CF_3$, $-NO_2$, -CN, $-CO_2H$, carboxy, alkenylamino, alkynylamino, di($C_1-C_3$ alkyl)amino, $-C(O)O-(C_1-C_3$ alkyl), $-C(O)NH-(C_1-C_3$ alkyl), -CH=NOH, $-PO_3H_2$, $-C(O)N(C_1-C_3$ alkyl$)_2$, haloalkyl, alkoxyalkoxy, alkoxyalkyl, alkoxycarbonyl, carboxaldehyde, carboxamide, cycloalkyl, cycloalkenyl, cycloalkynyl, cycloalkylalkyl, aryl, aroyl, aryloxy, arylamino, biaryl, thioaryl, diarylamino, heterocyclyl, alkylaryl, aralkenyl, aralkyl, alkylheterocyclyl, heterocyclylalkyl, heterocycloyl, sulfonyl, $-SO_2-(C_1-C_3$ alkyl), $-SO_3-(C_1-C_3$ alkyl), sulfonamido, aryloxyalkyl, carbamate and -C(O)NH(benzyl);

wherein $R^1$, $R^2$, $R^3$ and $R^4$ are unsubstituted or substituted with at least one electron donating or electron withdrawing group;

and pharmaceutically acceptable salts thereof.

Suitable derivatives, representative of general formula II are disclosed in U.S. Patent Nos. 5,166,172; 5,290,807; 5,180,738 and 5,164,410, hereby incorporated by reference.

A presently preferred fumagillol derivative is 6-O-(N-chloroacetylcarbamoyl) fumagillol, also known as TNP-470, of Formula III, shown below. The synthesis of this compound is disclosed in U.S. Patent No. 5,180,738, hereby incorporated by reference.

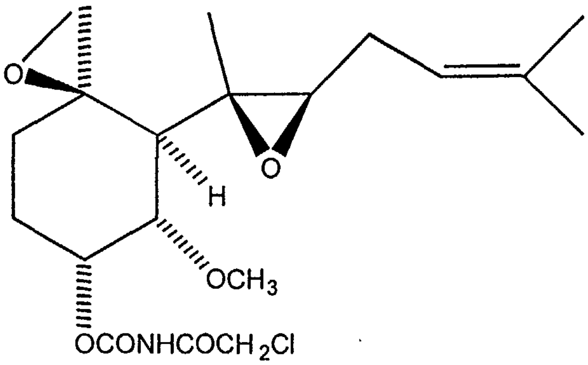

Formula III

As used herein, the phrase "antineoplastic agent" refers to a compound which prevents the development, maturation or spread of neoplastic (abnormal) cells. Suitable antineoplastic agents include paclitaxel, gemcitabine and carmustine (1,3-bis(2-chloroethyl)-1-nitrosourea); wherein paclitaxel is presently preferred. Combinations of antineoplastic agents may also be advantageous.

The active ingredient (components) of the present kits can be used in the form of the drug itself, or a pharmaceutically acceptable salt derived from inorganic or organic acids. The phrase "pharmaceutically acceptable salt" means those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts are well-known in the art. For example, S. M. Berge *et al.* describe pharmaceutically acceptable salts in detail in J. Pharmaceutical Sciences, 1977, 66: 1 *et seq.*

The salts can be prepared *in situ* during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphor sulfonate, digluconate, glycerophosphate, hemisulfate, heptanoate, hexanoate, fumarate, hydrochloride, hydrobromide, hydroiodide, 2-hydroxyethansulfonate (isothionate), lactate, maleate, methane sulfonate, nicotinate, 2-naphthalene sulfonate, oxalate, palmitoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

Basic addition salts can be prepared *in situ* during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylammonium, dimethylammonium, trimethylammonium, triethylammonium, diethylammonium, and ethylammonium among others. Other representative organic amines useful for the formation of base addition salts include ethylenediamine, ethanolamine, diethanolamine, piperidine, piperazine and the like.

The components of this invention may be administered to humans orally, rectally, parenterally, intracisternally, intravaginally, intraperitoneally, topically (as by powders, ointments or drops), bucally or as an oral or nasal spray; if appropriate for the particular drug selected. The term "parenterally," as used herein, refers to modes of administration which include intravenous, intramuscular, intraperitoneal, intrasternal, subcutaneous and intraarticular injection and infusion.

Dosage forms for topical or transdermal administration of the components of this invention include pastes, creams, lotions, gels, powders, solutions, sprays, ointments, patches or inhalants. The active compound is mixed under sterile conditions with a pharmaceutically acceptable carrier and any needed preservatives, buffers or propellants which

can be required. Opthalmic formulations, eye ointments, powders and solutions are also contemplated as being within the scope of this invention.

Components of the present invention may also be in a physiologically tolerable diluent. The present invention includes the components as described above formulated into compositions together with one or more non-toxic physiologically tolerable or acceptable diluents, carriers, adjuvants or vehicles that are collectively referred to herein as diluents, for parenteral injection, for intranasal delivery, for oral administration in solid or liquid form, for rectal or topical administration, or the like.

The compositions can also be delivered through a catheter for local delivery at a target site, via an intracoronary stent (a tubular device composed of a fine wire mesh), or via a biodegradable polymer. The components may also be complexed to ligands, such as antibodies, for targeted delivery.

Compositions suitable for parenteral injection may comprise physiologically acceptable, sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propylene glycol, polyethylene glycol, glycerol, and the like), vegetable oils (such as olive oil), sugars, cyclodextrins, injectable organic esters such as ethyl oleate, and suitable mixtures thereof.

These compositions can also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride and the like. Prolonged absorption of the injectable pharmaceutical form can be brought about by the use of agents delaying absorption, for example, aluminum monostearate and gelatin.

Suspensions, in addition to the active components, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Proper fluidity can be maintained, for example, by the use of coating materials such as lecithin, by the maintenance of the required particle size in the case of dispersions and by the use of surfactants.

In some cases, in order to prolong the effect of the drug, it is desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Solid dosage forms for oral administration include capsules, tablets, pills, powders and granules. In such solid dosage forms, the active component may be mixed with at least one inert, pharmaceutically acceptable excipient or carrier, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The active components can also be in micro-encapsulated form, if appropriate, with one or more of the above-mentioned excipients.

Alternatively, the components may be pressurized and contain a compressed gas, such as nitrogen or a liquified gas propellant. The liquified propellant medium and indeed the total composition is preferably such that the active ingredient does not dissolve therein to any substantial extent. The pressurized composition may also contain a surface active agent. The surface active agent may be a liquid or solid non-ionic surface active agent or may be a solid anionic surface active agent. It is preferred to use the solid anionic surface active agent in the form of a sodium salt.

Transdermal patches may also be used to provide controlled delivery of an active component to the body.

Such dosage forms can be made by dissolving or dispensing the component in the proper medium. Absorption enhancers can also be used to increase the flux of the compound across the skin. The rate can be controlled by either providing a rate-controlling membrane or by dispersing the compound in a polymer matrix or gel.

A further form of topical administration is to the eye, as for the treatment of immune-mediated conditions of the eye such as autoimmune diseases, allergic or inflammatory conditions, and corneal transplants. Components of the invention may be delivered in a pharmaceutically acceptable ophthalmic vehicle, such that the component is maintained in contact with the ocular surface for a sufficient time period to allow the component to penetrate the corneal and internal regions of the eye, as for example the anterior chamber, posterior chamber, vitreous body, aqueous humor, vitreous humor, cornea, iris/cilary, lens, choroid/retina and sclera. The pharmaceutically acceptable ophthalmic vehicle may, for example, be an ointment, vegetable oil or an encapsulating material.

Liquid dosage forms for oral administration include pharmaceutically acceptable emulsions, solutions, suspensions, syrups and elixirs. In addition to the active components, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Compositions for rectal or vaginal administration are preferably suppositories which can be prepared by mixing the compounds of this invention with suitable non-irritating excipients or carriers such as cocoa butter, polyethylene glycol or a suppository wax which are solid at room temperature but liquid at body temperature and therefore melt in the rectum or vaginal cavity and release the active component.

Components of the present invention may also be administered in the form of liposomes. As is known in the art, liposomes are generally derived from phospholipids or other lipid substances. Liposomes are formed by mono-or multi-lamellar hydrated liquid crystals which are dispersed in an aqueous medium. Any non-toxic, physiologically acceptable and metabolizable lipid capable of forming liposomes can be used. The present components in liposome form can also contain stabilizers, preservatives, excipients and the like. The preferred lipids are natural and synthetic phospholipids and phosphatidyl cholines (lecithins) used separately or together.

Methods to form liposomes are known in the art. See, for example, Prescott, Ed., Methods in Cell Biology, Volume XIV, Academic Press, New York, N.Y. (1976), p. 33 *et seq.*

*Three-Component Kits*

Another kit of the present invention includes at least three components: a fumagillol derivative, at least one antineoplastic agent and at least one platinum complex.

Appropriate fumagillol derivatives and antineoplastic agents are as described above. Moreover, appropriate dosage forms are also as described above.

The ratio of fumagillol to antineoplastic agent is not critical, and will depend upon the particular patient and tumor to be treated.

As used herein, the phrase "platinum complex" refers to compounds in which platinum is complexed. Examples of platinum complexes include cisplatin, carboplatin, nedaplatin, zeniplatin, enloplatin, lobaplatin, ormaplatin, oxaliplatin, loboplatin and sebriplatin, wherein carboplatin is a presently preferred platinum complex. It may be advantageous to use more than one platinum complex.

*The Methods*

Actual dosage levels of active ingredients in the pharmaceutical compositions of this invention can be varied so as to obtain an amount of the active compound(s) which is effective to achieve the desired therapeutic response for a particular patient. The selected dosage level will depend upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved.

The phrase "therapeutically effective amount" of the compound of the invention means a sufficient amount of the compound to treat disorders, at a reasonable benefit/risk ratio applicable to any medical treatment. It will be understood, however, that the total daily usage of the compounds and compositions of the present invention will be decided by the attending physician within the scope of sound medical judgement.

The specific therapeutically effective dose level for any particular patient will depend upon a variety of factors including the disorder being treated and the severity of the disorder; activity of the specific compound employed; the

specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to either start doses of the compound at levels lower than required to achieve the desired therapeutic effect and to gradually increase the dosage until the desired effect is achieved, or start doses of the compound at high levels and to gradually decrease the dosage until the desired effect is achieved, as appropriate for the care of the individual patient.

In addition, the amount of each component to be administered also depends upon the route of administration, such as whether administration is continuous (via continuous infusion pump); once a week; or more than once a week.

TNP-470 administration will be described in greater detail as a representative example of the administration procedures for fumagillol derivatives in general. For 6-O-(N-chloroacetylcarbamoyl)fumagillol (TNP-470), the following information may serve as a general guideline for administration. If TNP-470 is administered several times a week, it may be administered in an amount of from about 20 to about 180 $mg/m^2$ /day; preferably in an amount of from about 25 to about 100 $mg/m^2$ /day; and most preferably in an amount of from about 30 to about 60 $mg/m^2$ /day. Usually, the compound is administered from three to five times a week, if it is to be plurally administered in a given week.

If TNP-470 is administered once a week, it may be administered in an amount of from about 20 to about 200 $mg/m^2$/week; preferably in an amount of from about 40 to about 180 $mg/m^2$/week; and most preferably in an amount of from about 135 to about 175 $mg/m^2$/week.

If TNP-470 is administered continuously (via continuous infusion pump), it may be administered in an amount of from about 1 to about 10 $mg/m^2$ /day; preferably in an amount of from about 1.25 to about 5 $mg/m^2$ /day; and most preferably in an amount of from about 1 to about 3 $mg/m^2$/day. For continuous administration, the component is usually administered for at least five consecutive days of the week.

Paclitaxel administration will be described in greater detail as a representative example of the administration procedures for anteneoplastic agents in general. For paclitaxel (TAXOL), the following information may serve as a general guideline for administration. If paclitaxel is administered once a week, it may be administered in an amount of from about 50 to about 125 $mg/m^2$/week; preferably in an amount of from about 75 to about 105 $mg/m^2$ /week; and most preferably in an amount of from about 90 to about 100 $mg/m^2$ /week.

If paclitaxel is administered once every three weeks, it may be administered in an amount of from about 135 to about 250 $mg/m^2$ /3 weeks; preferably in an amount of from about 175 to about 230 $mg/m^2$ /3 weeks; and most preferably in an amount of from about 175 to about 225 $mg/m^2$/3 weeks.

Paclitaxel is usually administered intravenously, by introducing the drug over a one to three hour period. However, continuous infusion may also be used. Normally, paclitaxel is administered before TNP-470, although the order may be reversed. Furthermore, paclitaxel administration may begin at any point before or after initiating TNP-470 administration, but should not be administered through the same intravenous line as TNP-470; and paclitaxel is most often given immediately before initiating the next dose of TNP-470. In conjunction with paclitaxel administration, antiemetic therapy and prophylaxis against paclitaxel hypersensitivity may be administered according to standard medical practice.

Carboplatin administration will be described in greater detail as a representative example of the administration procedures for platinum complexes in general. For carboplatin (PARAPLATIN), the following information may serve as a general guideline for administration. Carboplatin is usually administered once every three weeks, so it may be administered in an amount of from about AUC 5 to about AUC 6/3 weeks and most preferably in an amount of from about AUC 6 /3 weeks.

When three components are to be administered, paclitaxel is preferably administered before TNP-470 and carboplatin after, although order of administration is not critical.

When the fumagillol derivative is treated in conjunction with treatment of an antineoplastic agent, in a two-component regimen, each component is usually administered sequentially. Each component may be administered in plural administrations, or as a single dose. Moreover, each component may be continuously administered. In addition to the presently preferred sequential administration, other regimens for the treatment include: (1) administering the fumagillol derivative and the antineoplastic agent in plural administrations simultaneously and intermittently; (2) administering the fumagillol derivative and the antineoplastic agent simultaneously, each in a single dose; (4) administering the fumagillol derivative and the antineoplastic agent simultaneously and continuously; and (5) administering the fumagillol derivative is intermittantly and administering the antineoplastic agent intermittantly.

The following is a specific exemplary regimen for administration of the two-component system.

Single doses of each component may be administered once a week in the following manner. Once a week, 50-100 $mg/m^2$ paclitaxel may be administered intravenously over an hour, followed by 40-180 $mg/m^2$ TNP-470 administered intravenously over four hours.

When the fumagillol derivative is administered in conjunction with treatment of an antineoplastic agent and a platinum complex, in a three-component regime, each component is usually administered sequentially. Each component may be administered in plural administrations, or as a single dose. Moreover, each component may be continuously administered. In addition to the presently preferred sequential administration, other regimens for the treatment include: (1) administering the fumagillol derivative, the platinum complex and the antineoplastic agent plural administrations simultaneously and intermittently; (2) administering the fumagillol derivative, the platinum complex and the antineoplastic agent simultaneously, each in a single dose; (3) administering the fumagillol derivative, the platinum complex and the antineoplastic agent simultaneously and continuously; (4) administering the fumagillol derivative intermittently, the platinum complex intermittently and the antineoplastic agent intermittently; (5) administering the fumagillol continuously, the platinum complex intermittently and the antineoplastic agent intermittently; and (6) administering the drugs in a three-week cycle wherein the antineoplastic agent is administered continuously or intermittently during the first week, the fumagillol derivative is administered continuously or intermittently during the second week, and the platinum complex is administered continuously or intermittently during the third week.

The following is a specific exemplary regimen for administration of the three-component system.

Paclitaxel is administered intravenously over three hours at doses of 90 to 100 mg/m$^2$ once a week, TNP-470 is administered continuously intravenously via continuous infusion pump at 2.5 mg/m$^2$/day at least five days of every seven days; and carboplatin is administered at a dose calculated to provide AUC exposure of 5-6 mg/mL/min. once a week, after paclitaxel but before TNP-470.

Another exemplary regimen regimen for administration of the three-component system is as follows.

Every three weeks, as the patient's condition permits, paclitaxel is administered intravenously over three hours at doses of 200 to 225 mg/m$^2$, followed immediately by 60 mg/m$^2$ TNP-470 administered over one hour, followed immediately by carboplatin administered intravenously at a dose calculated to provide AUC exposure of 5-6 mg/mL/min. Additional TNP-470 is then administered for five consecutive days out of every seven via continuous infusion pump at doses of 1 - 2.5 mg/m$^2$/day.

The following Examples are presented to describe preferred embodiments and utilities of the invention and are not meant to limit the invention unless otherwise stated in the claims appended hereto.

Example 1

To demonstrate the feasibility and effect of the treatment of human patients with TNP-470 combined with paclitaxel, the following Phase I clinical study was performed, using intermittent high doses of intravenous TNP-470 three times per week and a dose of intravenous paclitaxe every three weeks. Thirty two adult patients with advanced or metastatic cancer, including seventeen patients with lung cancer, were treated with TNP-470 (synthesized according to the procedure of U.S. Patent No. 5,180,738) in a liquid cyclodextrin-containing formulation using repeated administrations of high doses (20 - 60 mg/m$^2$) given over one hour, combined with 135-225 mg/m$^2$ paclitaxel given over three hours.

In this study, paclitaxel was administered first, followed one hour later by TNP-470. TNP-470 administrations were repeated up to three times per week. Paclitaxel administrations were repeated every three weeks as the patients' condition permitted. Anti-emetic therapy and prophylaxis against paclitaxel hypersensitivity were administered in accordance with the paclitaxel package insert on days when paclitaxel was administered.

The patients' tumors were measured at the start of the study, after six to eight weeks of therapy, and at two-month intervals thereafter. Patients continued to receive TNP-470 until tumors grew by 50% or they experienced adverse events. Patients received six to eight courses of paclitaxel, then continued TNP-470 alone without the cytocidal pharmaceutical if their tumors did not increase in size.

Patients with lung cancer experienced a greater than 70% increase in survival over that previously reported for patients treated with paclitaxel alone (14.3 months vs. 8 months). Detailed results are provided in Table 1 below, wherein row 1 reports results for paclitaxel alone, while rows 3 and 4 report results for this study.

In the table, "response" refers to the percentage of patients whose tumor shrank by more than fifty percent; and "time to progression" refers to the median value of the length of time between the first dose of drug and observation that the tumor has grown by more than fifty percent, or that new tumors have appeared. Patients who stop taking the drug for other reasons are considered lost to follow-up for "time to progression" calculations. "Survival" in the table is a median value.

Table 1

| TNP-470 dosage (mg/ $m^2$) | Cytotoxic therapy | Number/ type of patients | Responses (percentage) | Time to Progression (months) | Survival (months) |
|---|---|---|---|---|---|
| none | paclitaxel | lung cancer historical data | 20-30 | ~3 | 6-9 |
| none | paclitaxel and carboplatin | lung cancer historical data | 15-36 | ~4 | 8-9 |
| 20-60 | 135-225 mg/$m^2$ paclitaxel | 15 patients (lung cancer only) | 33 | 5.4 | 14.3 |
| 20-60 | 135-225 mg/$m^2$ paclitaxel | 32 patients (different cancers) | 28 | not applicable | not applicable |
| 60 | 225 mg/$m^2$ paclitaxel; carboplatin AUC 5 | 3 patients (lung cancer only) | 100 | 7, 8, 8 | 10, 16, 22+ |
| 60 | 225 mg/$m^2$ paclitaxel; carboplatin AUC 6 | 14 patients (different cancers) | 0 | regimen not tolerated | regimen not tolerated |
| 2.5-10 with or without 60 on Day 1 | 200-225 mg/$m^2$ paclitaxel; carboplatin AUC 5-6 | 20 patients (different cancers) | 20 | ongoing | ongoing |

Example 2

To demonstrate the effectiveness of the treatment of cancer patients with the combination of intermittent TNP-470 with paclitaxel and carboplatin, the following Phase I clinical sfudy was performed, according to the procedure described in Example 1 above, with the addition of carboplatin once every three weeks. Doses of TNP-470 and paclitaxel were modified as described below.

Twenty adult patients with advanced or metastatic cancer, were treated with TNP-470 (synthesized according to the procedure of U.S. Patent No. 5,180,738) in a liquid cyclodextrin-containing formulation using repeated administrations of a high dose (60 mg/$m^2$) given over one hour, combined with 225 mg/$m^2$ paclitaxel given over three hours. Carboplatin was given at doses calculated to provide an "area-under-the-curve (AUC)" exposure of 5 or 6 mg/mL/min (hereinafter referred to as "a dose of AUC 5" or "a dose of AUC 6"), according to the formula

$$\text{Dose (mg)} = \text{AUC} \times (\text{glomerular filtration rate} + 25)$$

as described in the package insert for carboplatin. Glomerular filtration rate was estimated by creatinine clearance, either measured or calculated from the patient's age, weight, gender and serum creatinine according to the Cockcroft-Gault equation

$$\text{Cr CL} = ((140\text{-age}) \times \text{weight (kg)}/72 \times \text{serum creatinine}) \times 1.0 \text{ if male or}$$

$$0.85 \text{ if female}$$

In this study, paclitaxel was administered first, followed one hour later by TNP-470, then finally by the carboplatin. TNP-470 administrations were repeated up to three thimes per week. Paclitaxel and carboplatin administrations were repeated every three weeks as the patients' condition permitted. Anti-emetic therapy and prophylaxis against paclitaxel hypersensitivity were administered in accordance with the paclitaxel package insert on days when paclitaxel

and carboplatin were administered.

The patients' tumors were measured at the start of the study, after six to eight weeks of therapy, and at two-month intervals thereafter. Patients continued to receive TNP-470 until tumors grew by 50% or they experienced adverse events. Patients received six to eight courses of paclitaxel and carboplatin, then continued TNP-470 treatment alone if their tumors did not increase in size. Patients with non-small-cell lung cancer experienced tumor shrinkage.

Three patients, all with non-small cell lung cancer, received TNP-470 in combination with paclitaxel and carboplatin (a dose of AUC 5). All three had partial responses and had survival often, sixteen and more than twenty-two months respectively (reported in row 5 of Table 1 above), compared to a reported median survival of only eight months for paclitaxel and carboplatin alone (reported in row 2 of Table 1 above). Patients receiving the higher carboplatin dose (a dose of AUC 6) represented a variety of different cancers, had received prior chemotherapy, and in general tolerated the combined therapy poorly beyond two cycles of therapy and thus did not receive sufficient therapy to evaluate effectiveness (reported in row 6 of Table 1 above) .

Example 3

To demonstrate the effectiveness of the treatment of cancer patients with the combination of continuously infused TNP-470 with paclitaxel and carboplatin, the following Phase I clinical study was performed, according to the procedure described in Example 1 above, with the following modifications.

Twenty adult patients with metastatic cancer, including patients with lung cancer, were treated with TNP-470, using repeated continuous administrations of low doses (2.5-10 mg/$m^2$ over five days in combination with paclitaxel at doses of 200-225 mg/$m^2$ and carboplatin at a dose of AUC 5 or AUC 6 (as explained in Example 2 above) reported in row 7 of Table 1 above.

In this study, paclitaxel was administered first, followed by the carboplatin, then by TNP-470 administered intravenously via portable continuous infusion syringe pump (available from Sims-Deltec) at a dose of 2.5 -10 mg/$m^2$ for five days (115-120 hours). The five-day TNP-470 infusion was repeated every week. Paclitaxel and carboplatin administrations were repeated every three weeks as the patient's condition permitted. Anti-emetic therapy and prophylaxis against paclitaxel hypersensitivity were administered in accordance with the paclitaxel package insert on days when paclitaxel and carboplatin were administered.

In an ongoing cohort of this study, in addition to the described therapy, nine patients received 60 mg/$m^2$ TNP-470 as a one-hour intravenous infusion after infusion of paclitaxel and prior to infusion of carboplatin. This high-dose one-hour TNP-470 infusion was administered only when paclitaxel and carboplatin were administered.

As in the prior experiments, the patient's tumors were measured at the start of the study, after six to eight weeks of therapy, and at two-month intervals thereafter. Patients continued to receive TNP-470 until tumors grew by 50% or they experienced adverse events. Patients received six to eight courses of paclitaxel and carboplatin, then continued TNP-470 alone if their tumors did not increase in size.

All references cited are hereby incorporated by reference.

The present invention is illustrated by way of the foregoing description and examples. The foregoing description is intended as a non-limiting illustration, since many variations will become apparent to those skilled in the art in view thereof. It is intended that all such variations within the scope and spirit of the appended claims be embraced thereby.

Changes can be made in the composition, operation and arrangement of the method of the present invention described herein without departing from the concept and scope of the invention as defined in the following claims:

## Claims

**1.** A method of treating a tumor comprising the step of administering a therapeutically-effective amount of

a fumagillol derivative; and,
at least one antineoplastic agent selected from the group consisting of paclitaxel, gemcitabine and carmustin;
to a patient in need of such treatment.

**2.** The method of claim 1 wherein said fumagillol derivative and said antineoplastic agent are administered sequentially.

**3.** The method of claim 1 wherein said fumagillol derivative is 6-O-(N-chloroacetylcarbamoyl) fumagillol and said antineoplastic agent is paclitaxel.

**4.** The method of claim 3 wherein 6-O-(N-chloroacetylcarbamoyl) fumagillol is administered three times a week and paclitaxel is administered once every three weeks.

**5.** The method of claim 4 wherein 30-60 mg/$m^2$ 6-O-(N-chloroacetylcarbamoyl) fumagillol is administered three times a week and 175-225 mg/$m^2$ paclitaxel is administered once every three weeks.

**6.** The method of claim 3 wherein 135-175 mg/$m^2$ 6-O-(N-chloroacetylcarbamoyl) fumagillol is administered once a week and 90-100 mg/$m^2$ paclitaxel is administered once a week.

**7.** A method of treating a tumor comprising the step of administering a therapeutically-effective amount of

a fumagillol derivative;
at least one antineoplastic agent selected from the group consisting of paclitaxel, gemcitabine and carmustine; and
at least one platinum complex;
to a patient in need of such treatment.

**8.** The method of claim 7 wherein said platinum complex is selected from the group consisting of cisplatin, carboplatin, nedaplatin, zeniplatin, enloplatin, lobaplatin, ormaplatin, oxaliplatin, loboplatin and sebriplatin.

**9.** The method of claim 7 wherein said fumagillol derivative, said platinum complex and said antineoplastic agent are administered sequentially.

**10.** The method of claim 7 wherein said antineoplastic agent is paclitaxel, said fumagillol derivative is 6-O-(N-chloroacetylcarbamoyl) fumagillol and said platinum complex is carboplatin.

**11.** The method of claim 10 for treating a tumor in a three-week cycle wherein paclitaxel is administered once every three weeks, 6-O-(N-chloroacetylcarbamoyl) fumagillol is administered three times a week, and carboplatin is administered once every three weeks.

**12.** The method of claim 11 for treating a tumor in a three-week cycle wherein 175-225 mg/$m^2$ paclitaxel is administered once every three weeks, 30-60 mg/$m^2$ 6-O-(N-chloroacetylcarbamoyl) fumagillol is administered three times a week, and carboplatin at a dose of AUC 5-6 is administered once every three weeks.

**13.** The method of claim 12 for treating a tumor in a three-week cycle wherein 225 mg/$m^2$ paclitaxel is administered once every three weeks, 60 mg/$m^2$ 6-O-(N-chloroacetylcarbamoyl) fumagillol is administered three times a week, and carboplatin at a dose of AUC 5 is administered once every three weeks.

**14.** The method of claim 10 for treating a tumor in a three-week cycle wherein 175-225 mg/$m^2$ paclitaxel is administered once every three weeks, 2.5-10 mg/$m^2$ 6-O-(N-chloroacetylcarbamoyl) fumagillol is administered continuously, and carboplatin at a dose of AUC 5-6 is administered once every three weeks.

**15.** The method of claim 14 for treating a tumor in a three-week cycle wherein 225 mg/$m^2$ paclitaxel is administered once every three weeks, 2.5-10 mg/$m^2$ 6-O-(N-chloroacetylcarbamoyl) fumagillol is administered continuously, and carboplatin at a dose of AUC 5 is administered once every three weeks.

**16.** A kit comprising:

a fumagillol derivative, optionally in a pharmaceutically acceptable carrier or excipient; and,
at least one antineoplastic agent selected from the group consisting of paclitaxel, gemcitabine and carmustin, optionally in a pharmaceutically acceptable carrier or excipient.

**17.** The kit of claim 16 wherein said fumagillol derivative is 6-O-(N-chloroacetylcarbamoyl) fumagillol and said antineoplastic agent is paclitaxel.

**18.** A kit comprising:

a fumagillol derivative, optionally in a pharmaceutically acceptable carrier or excipient;

at least one antineoplastic agent selected from the group consisting of paclitaxel, gemcitabine and carmustine, optionally in a pharmaceutically acceptable carrier or excipient; and
at least one platinum complex, optionally in a pharmaceutically acceptable carrier or excipient.

**19.** The kit of claim 18 wherein said platinum complex is selected from the group consisting of cisplatin, carboplatin, nedaplatin, zeniplatin, enloplatin, lobaplatin, ormaplatin, oxaliplatin, loboplatin and sebriplatin.

**20.** The kit of claim 18 wherein said antineoplastic agent is paclitaxel, said fumagillol derivative is 6-O-(N-chloroacetylcarbamoyl) fumagillol and said platinum complex is carboplatin.